# EUROPEAN PATENT SPECIFICATION

(11) **EP 1 541 157 B1**
(45) Date of publication and mention of the grant of the patent: **14.11.2012**
(21) Application number: 03795344.5
(22) Date of filing: 09.09.2003
(51) Int. Cl.: A61K 35/12, A61L 27/36

(54) **METHOD OF TREATING BIOLOGICAL TISSUE FOR TRANSPLANTATION BY APPLYING ULTRAHIGH HYDROSTATIC PRESSURE**
VERFAHREN ZUR BEHANDLUNG VON BIOLOGISCHEM GEWEBE ZUR TRANSPLANTATION DURCH AUFBRINGEN VON ULTRAHOHEM HYDROSTATISCHEM DRUCK
PROCEDE DE TRAITEMENT D'UN TISSU BIOLOGIQUE DESTINE A LA TRANSPLANTATION PAR APPLICATION D'UNE PRESSION HYDROSTATIQUE ULTRA-ELEVEE

(30) Priority: 10.09.2002 JP 2002264470
(43) Date of publication of application: 15.06.2005
(73) Proprietor: Japan as represented by president of National Cardiovascular Center, Suita-shi, Osaka 565-8565 (JP); NIPRO CORPORATION, Osaka-shi, Osaka 531-8510 (JP)
(72) Inventor: FUJISATO, Toshiya, National Cardiovascular Center, Suita-shi, Osaka 565-8565 (JP); KISHIDA, Akio, National Cardiovascular Center, Suita-shi, Osaka 565-8565 (JP); FUNAMOTO, Seiichi, National Cardiovascular Center, Suita-shi, Osaka 565-8565 (JP); NAKATANI, Takeshi, National Cardiovascular Center, Suita-shi, Osaka 565-8565 (JP); KITAMURA, Soichiro, National Cardiovascular Center, Suita-shi, Osaka 565-8565 (JP)
(74) Representative: Reitstötter - Kinzebach
(86) International application number: PCT/JP2003/011529
(87) International publication number: WO 2004/024170

(56) References cited:
- WO-A-93/12731
- WO-A1-01/82992
- WO-A1-02/49681
- WO-A1-98/04300
- WO-A1-99/41981
- WO-A2-02/14480
- WO-A2-02/40630
- BADER A ET AL: "Tissue engineering of heart valves--human endothelial cell seeding of detergent acellularized porcine valves." EUROPEAN JOURNAL OF CARDIO-THORACIC SURGERY : OFFICIAL JOURNAL OF THE EUROPEAN ASSOCIATION FOR CARDIO-THORACIC SURGERY SEP 1998, vol. 14, no. 3, September 1998 (1998-09), pages 279-284, XP002527247 ISSN: 1010-7940
- STEINHOFF GUSTAV ET AL: "Tissue engineering of pulmonary heart valves on allogenic acellular matrix conduits: In vivo restoration of valve tissue" CIRCULATION, vol. 102, no. 19 Supplement, 7 November 2000 (2000-11-07), pages III.50-III.55, XP002527437 ISSN: 0009-7322
- SODIAN R ET AL: "Early in vivo experience with tissue-engineered trleaflet heart valves" CIRCULATION, LIPPINCOTT WILLIAMS & WILKINS, US, vol. 102, no. SUPPL. III, 7 November 2000 (2000-11-07), pages II-22, XP002959988 ISSN: 0009-7322
- TOSHIYA FUJISATO ET AL.: 'Saisei iryoyo seitai yurai sozai no rikigaku tokusei' THE JAPAN SOCIETY OF MECHANICAL ENGINEERS 2002 NENJI TAIKAI KOEN RONBUNSHU vol. 6, 20 September 2002, pages 47 - 48, XP002973840
- TOSHIYA FUJISATO ET AL.: 'Buta shinzoben no datsusaiboka shori to seitai rikigaku tokusei' THE JAPAN SOCIETY OF MECHANICAL ENGINEERS DAI 12 KAI BIOENGINEERING GAKUJUTSU KOENKAI.SHUKI SEMINAR KOEN RONBUNSHU 2001, pages 199 - 200, XP002973841
- TOSHIYA FUJISATO ET AL.: 'Seitai scaffold to shite no datsusaiboka buta shinzoben' THE JAPAN SOCIETY OF MECHANICAL ENGINEERS DAI 14 KAI BIOENGINEERING GAKUJUTSU KOENKAI.SHUKI SEMINAR KOEN RONBUNSHU March 2002, pages 41 - 42, XP002973842

## Description

### Field of The Invention:

The present invention relates to decellularization and virus inactivation of native tissues from a donor for use in transplantation.

### Background Art:

Scafold materials are prepared from native tissues for clinical application by chemically treating the tissue with a fixing agent such as glutaraldehyde or by decellularizing the tissue. In the heart valve replacement, for example, xenogenic heart valves are prepared from porcine heart valves or bovine pericardia by treating with glutaraldehyde to diminish their immunogenicity. These xenogenic valves are well anti-clotting but durable only for 5-10 years in young recipients. Therefore, they are normally transplanted to old recipients over 60 years old.

Since tissue bank systems have been organized in Europe and America around 1985 and also in Japan in recent years, allogenic cryopreserved valves from deceased donors have been clinically used. The allogenic valves are less thrombogenic than mechanical valves, more durable than xenogenic valves and less susceptible to infections than both. However, a critical problem is the fact that the number of available valves is absolutely insufficient. Moreover, cases in which functional failure appeared at relatively early stage have been reported among young recipients suggesting the involvement of immune reactions. In Ross operation known to be effective in young recipients, autologous pulmonary valve is transplanted to aortic valve site and the impaired pulmonary valve is reconstructed with cryopreserved allogenic valve. The characteristic feature of the autologous pulmonary valve transplanted to the aortic valve site is that it is growable as the recipient grows. In contrast, mechanical valves and xenogenic valves as well as cryopreserved allogenic valves are not growable and re-transplatation is often needed for children. In order to eliminate the above problems, several studies have been reported to remove donor cells from allogenic valves so that their immunogenicity and involvement of immune reactions are diminished to increase the durability and autogenesis of transplanted valves.

A decellularization method using a chemical solution called "SynerGraft" was developed by CryoLife, U.S.A. It was reported that the decellularized tissue by this method was infiltrated into autologous cellular structure within several months and recellularized with autologous cells.

Harverich et al. of Hannover University, School of Medicine, Germany published a decellularization method using a detergent Triton X-100 and proteolytic enzyme tripsin solutions.

However, washing with detergent or other chemical solutions alone is not sufficiently effective to remove bacteria, viruses and other contaminants from the interior of tissue because the washing depends on diffusion and penetration of the washing solution from surfaces of the tissue. Because of these limitations, complete decellularization and removal of bacteria and viruses are hardly possible for large tissue materials. In order to achieve satisfactory effects by the chemical washing, it is necessary to increase the degree of treatment. This may lead to problems of post-graft calcification and removal of residual treating chemicals. As evidenced from BSE and CJD infections in the dura transplantation, safety assurance is very important for the tissue to be transplanted. Currently known treating processes do not assure complete inactivation of viral contaminants and infection incidents may often occur from the transplanted tissue contaminated with viruses.

### Disclosure of The Invention:

It is, therefore, an object of this invention to provide a method which can eliminate or ameliorate the disadvantages of prior art, namely the method can accomplish, first, removal of cellular components, bacteria and viruses from large size tissues, second, treatment without impairing the biomechanical properties of the tissue, and, thirdly, sterilization of the tissue in a simple manner in a short period of time.

As a result of intensive studies, we have developed a method of treating biological tissues for transplantation. The method has its basis on a discovery that destruction of tissue cells and bacteria as well as inactivation of viruses occur by applying ultrahigh hydrostatic pressure to biological tissues.

The present invention provides, therefore, a method of treating biological tissue for transplantation comprising applying ultrahigh hydrostatic pressure between 5,000 and 15,000 atms to a transplatable native tissue of mammalian origin in a liquid medium whereby the connective tissue thereof is rendered isolatable from the remainder of said native tissue including cell components while keeping intact the structure and biomechanical properties thereof.

### Brief Description of The Accompanying Drawings:

Fig. 1 is a microscopic view of porcine heart valve specimens taken in cross-section. The specimen treated with a detergent for 24 hours is shown in the left while the specimen treated under ultrahigh hydrostatic pressure of 10,000 atms for 10 minutes is shown in the right. Residual nuclei are observed in the interior of the detergent-treated tissue (lower left);

Fig. 2 is a similar view to Fig. 1. It is seen from the photographs that collagenous tissue is well preserved without loosening in both specimens treated with the detergent or ultrahigh hydrostatic pressure;

Fig. 3 is a graph of breaking strength of porcine valve leaflet specimens. The strength of the specimen treated with the detergent is shown in the left while the strength of the specimen treated at a ultrahigh hydrostatic pressure of 10,000 atms is shown in the right. The breaking strength increases as the treating time increases in the detergent treatment while the breaking strength substantially remains constant as the treating time increases in the treatment under ultrahigh hydrostatic pressure;

Fig. 4 is a graph showing elastic modulus of porcine valve leaflet specimens which were treated differently; and

Fig. 5 is a photograph showing the bacteriocidal effect of the application of ultrahigh hydrostatic pressure.

### Best Mode For Carrying Out The Invention:

The term "ultrahigh hydrostatic pressure" as used herein refers to a pressure from 5,000 to 15,000 atms . The ultrahigh hydrostatic pressure has ever been studied for bacteria destruction in the field of food industry. However, its application to the preparation of transplantable tissues are not known.

Considering various factors affecting satisfactory results, we have developed a new method for treating native tissues for use in transplantation by setting a suitable range of treating conditions including treating temperature. Ultrahigh hydrostatic pressure between 5,000 and 15,000 atms may be applied to the native biological tissue using any known apparatus such as Dr. CHEF available form Kobe Steel, Ltd. or ultrahigh apparatus for laboratory use available from Hikari Kogaku Co., Ltd. The apparatus includes a small chamber in which an ultrahigh hydrostatic pressure up to about 15,000 atms can be generated. The tissue to be treated is placed in the chamber and the ultrahigh pressure is applied to the tissue via a liquid medium. The temperature within the chamber may be controlled by regulating the temperature of the chamber mantle. Different from the treatment of foods, it is imperative for the treatment of biological tissues for transplantation purposes to retain their biomechanical properties by preventing denaturing of their matrix components. Consequently, a temperature below 0°C or above 40°C should be avoided during the application of ultrahigh hydrostatic pressure.

The treating time may be as short as 30 minutes in case of the ultrahigh pressure treatment to achieve complete destruction and removal of cells from the tissue. In contrast, a much longer time is required for the chemical washing method because removal of nuclei depends on the diffusion and penetration of a detergent from the tissue surface. Moreover, the application of ultrahigh pressure allows the treatment of tissue pieces uniformly even in deep portions thereof.

The method according to the present invention finds use in the following applications.

### 1. Soft mammalian tissues for transplantation

Decellularization of soft tissues from donors of cerebral or heart failure death or xenogenic porcine or bovine soft tissues for transplantation purposes may be mentioned. The efficiency of the subsequent step of washing out of destructed cells is greatly enhanced. At the same time, the immunogenecity of the tissue is largely diminished.

### 2. Hard mammarian tissues for transplantation

Similar to the soft mammarian tissues, hard tissues such as bone, cartilage or teeth may be treated for transplantation purposes.

### 3. Treatment of other biological tissues for medical use

Tissues of animal or plant origin including cells may be treated for the purpose of destructing cells.

### Example:

Fresh porcine hearts were purchased from a breeding farm and transported at 4°C. The warm ischemic time during the tissue isolation was controlled within 20 minutes. Pulmonary valves and aortas were excised and washed with Hank's solution and treated in an ultrahigh pressure apparatus available from Kobe Steel Ltd. under the name of Dr. CHEF to apply an ultrahigh hydrostatic pressure between 1,000 to 10,000 atms for 10 to 30 minutes. After treating the tissue was washed with PBS to remove cell residues. Specimens of the decellularized tissue were stained with HE and elastica-van Gieson staining and histologically evaluated by the microscopic observation.

The biomechanical properties of the decellularized tissue were evaluated using a specimen of the decellularized heart valve leaflet cut into a size of about 3 mm width and about 15 mm length to measure the breaking strength using a conventional tester (tensilon tester). The modulus of elasticity was calculated from the strain at the breaking point and the thickness of the leaflet calculated from the weight and specific gravity thereof. The disinfectivity of the treated vascular tissue was evaluated by inoculating with normal bacteria floras before treating and culturing the tissue after the treatment.

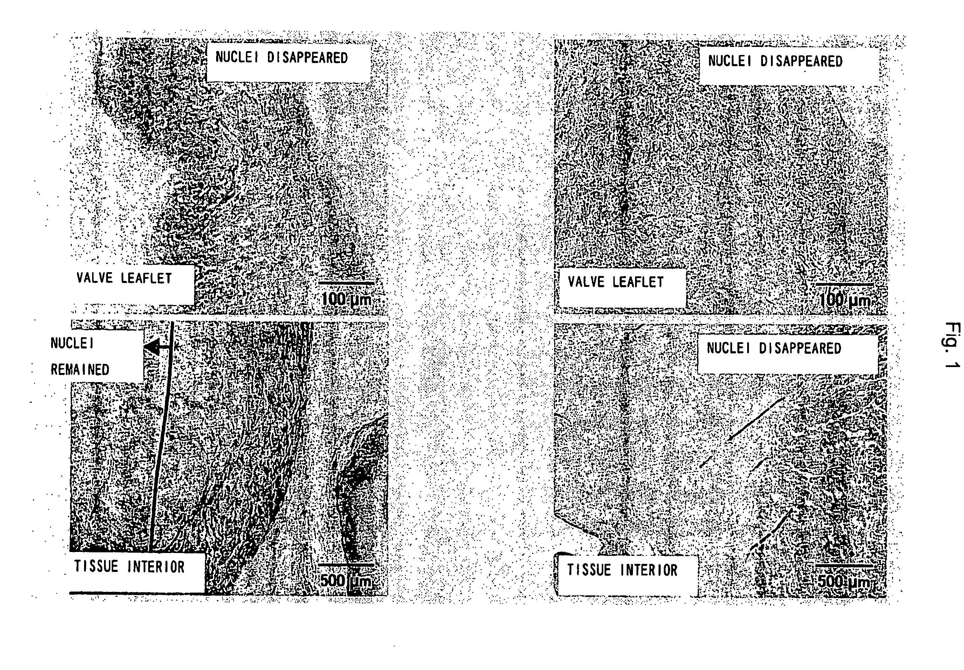

As shown in Fig. 1, the porcine pulmonary valve tissue was completely decellularized even in deep interior portions by the application of ultrahigh hydrostatic pressure according to the present invention. In contrast, the pulmonary valve leaflet treated with a detergent did not show stained nuclei in the tissue of several handreds µm thickness after immersing for 6 hours while the nuclei present in portions of 1 mm depth or more from the surface in the myocardium area of the leaflet base were stained after immersing for 24 hours.

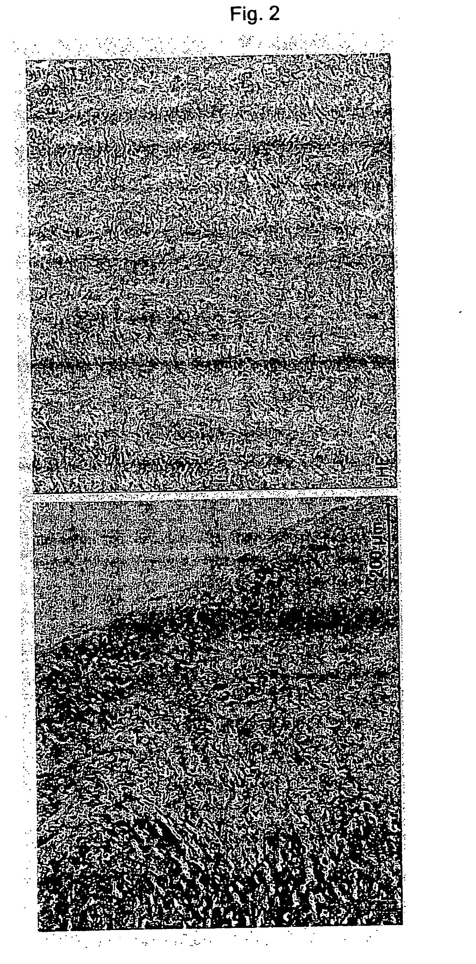

As shown in Fig. 2, collagen and elastin fibers were well maintained in the valve leaflet after applying the ultrahigh hydrostatic pressure according to the present invention.

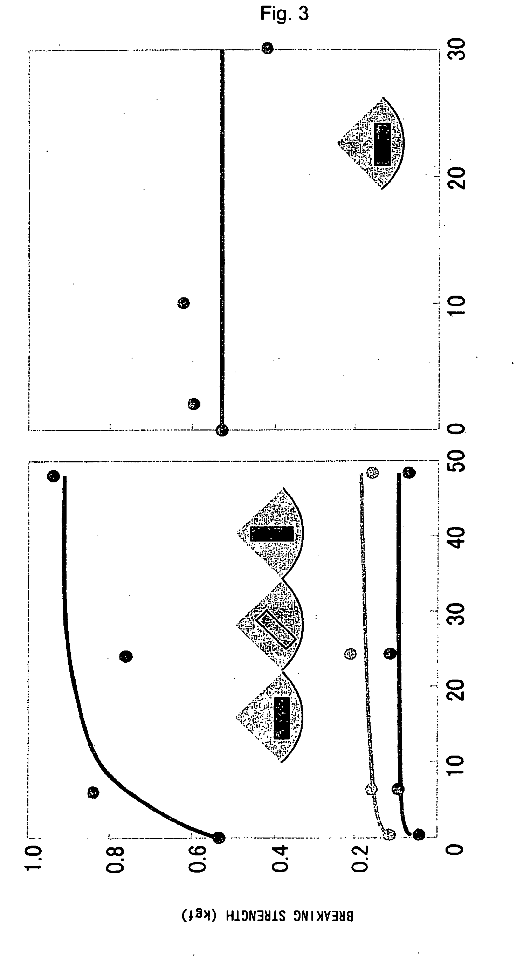

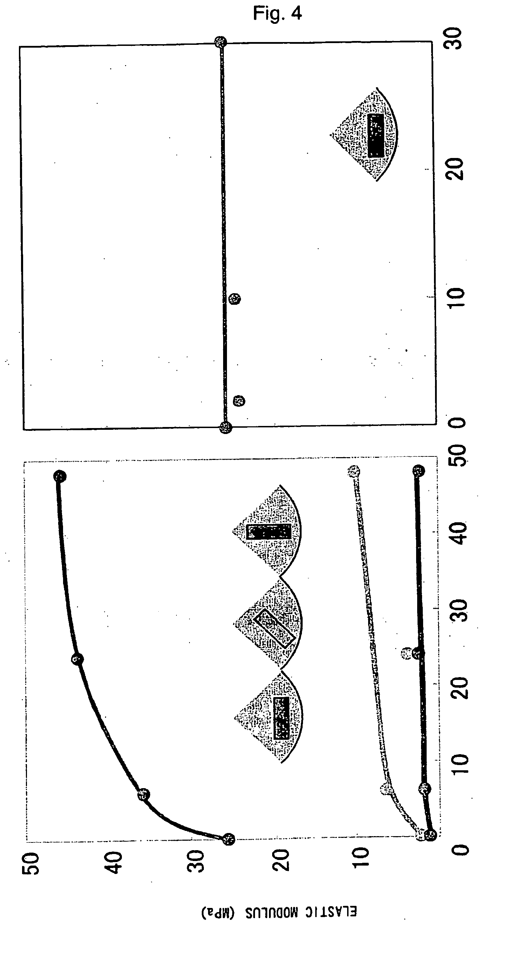

The specific gravity of normal porcine aortic leaflet as well as vascular walls was about 1.05. Studies on the anisotropy of biomechanical properties of native tissue revealed that the leaflet was flexible and weak in the direction of perpendicular to the direction tangent to the vascular walls of the leaflet and hard and strong in the direction parallel to the vascular walls. As shown in Figs. 3 and 4, the decellularization under the ultrahigh hydrostatic pressure according to the present invention did not significantly affect the biomechanical properties. The decellularization with a detergent solution showed a tendency of increasing both breaking strength and modulus of elasticity as the treating time increases.

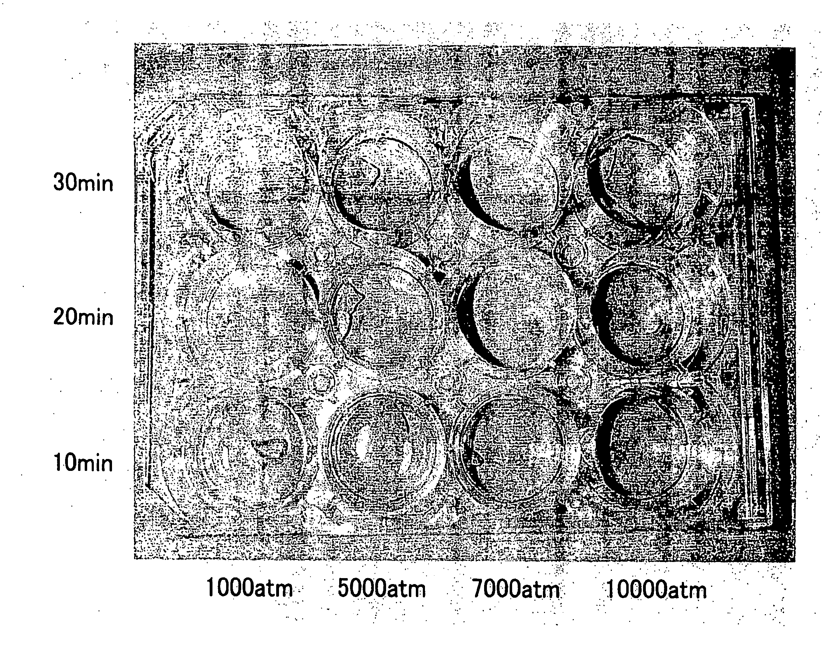

As shown in Fig. 5, the aortic tissue contaminated with normal bacteria floras before treatment was disinfected by the application of an ultrahigh hydrostatic pressure above 5,000 atms.

## Claims

1. A method of treating native tissue of mammalian origin for transplantation comprising applying ultrahigh hydrostatic pressure between 5,000 and 15,000 atms (approximately between 5.10⁸ pascal and 15.10⁸ pascal) to the tissue in a liquid medium whereby the connective tissue thereof is rendered isolatable from the remainder of said native tissue including cell components while keeping intact the structure and biomechanical, properties thereof.

2. The method of claim 1 wherein the native tissue to be treated includes vascular vessels, heart valves, cornea, amnion, dura and other mammalian tissues.

3. The method of claim 1 wherein the native tissue to be treated includes bone, cartilage, teeth or other hard mammalian tissues.

4. The method of claim 1 wherein the native tissue to be treated includes heart, kidney, lever, pancreas, bone, brain, other organs and part thereof.

5. The method of claim 1 wherein the ultrahigh hydrostatic pressure is applied at a temperature between 0°C and 40°C.

6. The method claim 1 wherein said liquid medium is purified water, a hypertonic solution, a hypotonic solution, a detergent solution, an enzyme solution

7. The method of claim 1 further comprising the step of removing destructed cells from the tissue by washing,

## Patentansprüche

1. Verfahren zur Behandlung nativen Säugetiergewebes zur Transplantation umfassend eine hydrostatische Ultrahochdruckbehandlung des Gewebes bei zwischen 5.000 und 15.000 atm (etwa zwischen 5 • 10⁸ Pascal und 15 • 10⁸ Pascal) in einem Flüssigmedium, wodurch dessen Bindegewebe vom Rest des nativen Gewebes, einschließlich den Zellkomponenten, unter Erhalt von dessen Struktur und biomechanischen Eigenschaften abgetrennt werden kann.

2. Verfahren nach Anspruch 1, wobei das zu behandelnde native Gewebe unter Gefäßen, Herzklappen, Kornea, Amnion, Dura und anderen Säugetiergeweben ausgewählt ist.

3. Verfahren nach Anspruch 1, wobei das zu behandelnde native Gewebe unter Knochen, Knorpel, Zähnen und anderen festen Säugetiergeweben ausgewählt ist.

4. Verfahren nach Anspruch 1, wobei zu behandelnde native Gewebe unter Herz, Niere, Leber, Pankreas, Knochen, Hirn, anderen Organen und deren Teilen ausgewählt ist.

5. Verfahren nach Anspruch 1, wobei die hydrostatische Ultrahochdruckbehandlung bei einer Temperatur zwischen 0°C und 40°C stattfindet.

6. Verfahren nach Anspruch 1, wobei das Flüssigmedium gereinigtes Wasser, eine hypertonische Lösung, eine hypotonische Lösung, eine Detergenslösung oder eine Enzymlösung ist.

7. Verfahren nach Anspruch 1, ferner umfassend den Schritt der Entfernung zerstörter Zellen aus dem Gewebe mittels Waschen.

## Revendications

1. Procédé de traitement d'un tissu natif d'origine mammalienne destiné à la transplantation, qui comprend d'appliquer une pression hydrostatique ultra-élevée comprise entre 5 000 et 15 000 atm (approximativement entre 5·10⁸ pascals et 15·10⁸ pascals) au tissu dans un milieu liquide, moyennant quoi le tissu conjonctif de celui-ci peut être isolé du reste dudit tissu natif comprenant des composants cellulaires tout en maintenant intactes la structure et les propriétés biomécaniques de celui-ci.

2. Procédé selon la revendication 1, dans lequel le tissu natif devant être traité comprend des vaisseaux vasculaires, des valves cardiaques, la cornée, l'amnios, la dure-mère et d'autres tissus de mammifères.

3. Procédé selon la revendication 1, dans lequel le tissu natif devant être traité comprend de l'os, du cartilage, des dents ou d'autres tissus durs de mammifères.

4. Procédé selon la revendication 1, dans lequel le tissu natif devant être traité comprend le coeur, le rein, le foie, le pancréas, l'os, le cerveau, d'autres organes et des parties de ceux-ci.

5. Procédé selon la revendication 1, dans lequel la pression hydrostatique ultra-élevée est appliquée à une température comprise entre 0 °C et 40 °C.

6. Procédé selon la revendication 1, dans lequel ledit milieu liquide est de l'eau purifiée, une solution hypertonique, une solution hypotonique, une solution détergente, une solution enzymatique.

7. Procédé selon la revendication 1, comprenant en outre l'étape qui comprend d'éliminer les cellules détruites du tissu par lavage.
